# EUROPEAN PATENT SPECIFICATION

(11) **EP 3 218 017 B1**
(45) Date of publication and mention of the grant of the patent: **02.11.2022**
(21) Application number: 15858561.2
(22) Date of filing: 10.11.2015
(51) Int. Cl.: A61L 2/10, A61L 2/24, A61L 2/28

(54) **USE OF A DECONTAMINATION APPARATUS**
VERWENDUNG EINES DEKONTAMINATIONSGERÄTS
UTILISATION D'UN APPAREIL DE DÉCONTAMINATION

(30) Priority: 10.11.2014 US 201462077512 P
(43) Date of publication of application: 20.09.2017
(60) Divisional application: 22172891.8
(73) Proprietor: Diversey, Inc., Fort Mill, SC 29708 (US)
(72) Inventor: DAYTON, Roderick M., Strongsville, OH 44136 (US)
(74) Representative: PGA S.p.A., Milano, Succursale di Lugano
(86) International application number: PCT/US2015/060047
(87) International publication number: WO 2016/077403

(56) References cited:
- EP-A1- 2 772 272
- US-A1- 2004 244 138
- US-A1- 2005 022 330
- US-A1- 2011 004 342
- US-A1- 2012 223 216
- US-A1- 2013 270 459
- US-A1- 2014 241 941
- US-B2- 8 516 651

## Description

### BACKGROUND OF THE INVENTION

### 1. Field of the Invention

This disclosure relates generally to use of an apparatus for decontaminating a plurality of surfaces, and optionally gathering information on ultraviolet energy at a variety of physical locations in a given environment and providing that information to a collection point for graphic display, analysis, and recording.

### 2. Description of Related Art

High touch environmental surfaces in healthcare facilities are recognized as significant sources of pathogens. To avoid exposing patients in such environments to infectious organisms, medical personnel working therein are required to take precautionary measures to disinfect high touch surfaces. One such measure is to expose entire rooms, in which the high touch surfaces reside, to disinfection technologies that employ high doses of ultraviolet light in the C spectrum, UVC. These high doses may be from continuous or pulsed sources, but one challenge with these technologies is to ensure that substantially all surfaces are suitably exposed to the UVC to achieve the desired level of pathogen reduction.

Decontamination apparatuses based on the emission of UVC light are e.g. known from US 2014/241941 and US 2014/330452

### BRIEF SUMMARY OF THE INVENTION

The invention relates to use of a decontamination apparatus as recited in the appended claims.

### BRIEF DESCRIPTION OF SEVERAL VIEWS OF THE DRAWING

The invention may take physical form in certain parts and arrangement of parts, embodiments of which will be described in detail in this specification and illustrated in the accompanying drawings which form a part hereof and wherein:
FIG. 1 shows a perspective view of an illustrative embodiment of a UVC sensor;
FIG. 2 shows a block diagram of the sensor operation;
FIG. 3 shows a graphic representation of the central collection point display and representative data fields;
FIG. 4 shows a flow diagram graphically illustrating the connectivity and action between the sensors and the central collection point;
FIG. 5 shows a perspective view of an autonomously-movable UVC source;
FIG. 6 shows a partially-cutaway view of a base of the UVC source shown in FIG. 5;
FIG. 7 shows an embodiment of a UVC source comprising a plurality of independently-adjustable UVC bulbs supported by a motorized base;
FIG. 8 shows a bottom view of one of the UVC bulbs shown in FIG. 7; and
FIG. 9 shows a flow diagram schematically illustrating a method of performing a decontamination process.

### DETAILED DESCRIPTION OF THE INVENTION

Certain terminology is used herein for convenience only and is not to be taken as a limitation on the present invention. Relative language used herein is best understood with reference to the drawings, in which like numerals are used to identify like or similar items. Further, in the drawings, certain features may be shown in somewhat schematic form.

It is also to be noted that the phrase "at least one of', if used herein, followed by a plurality of members herein means one of the members, or a combination of more than one of the members. For example, the phrase "at least one of a first widget and a second widget" means in the present application: the first widget, the second widget, or the first widget and the second widget. Likewise, "at least one of a first widget, a second widget and a third widget" means in the present application: the first widget, the second widget, the third widget, the first widget and the second widget, the first widget and the third widget, the second widget and the third widget, or the first widget and the second widget and the third widget.

In order to disinfect surfaces in healthcare facilities (e.g., beds in patient rooms, tray tables, seats, etc.), high doses of UVC are provided from a central source during an irradiation process. Currently, irradiation protocols must be tested to ensure that each part of the area is sufficiently disinfected. However, various layouts of rooms and shapes of furniture can make it difficult to expose each surface to be decontaminated to an adequate level of UVC to achieve the desired level of pathogen reduction. This often requires manually positioning a centrally-located, high-power UVC source at a desired location in the room, activating the UVC source to perform irradiation at one location, once irradiation is complete and the source deactivated, manually moving the source, and once again activating the source to irradiate again. This process is often repeated multiple times to ensure sufficient exposure of all surfaces to the UVC light, but is labor intensive and time consuming, rendering such protocols impractical. Thus, the present disclosure is directed to a system, apparatus, and method for sequentially exposing various different surfaces to UVC light, and optionally gathering information on ultraviolet energy at a variety of physical locations in a given environment and providing that information to a collection point for graphic display, analysis, and recording.

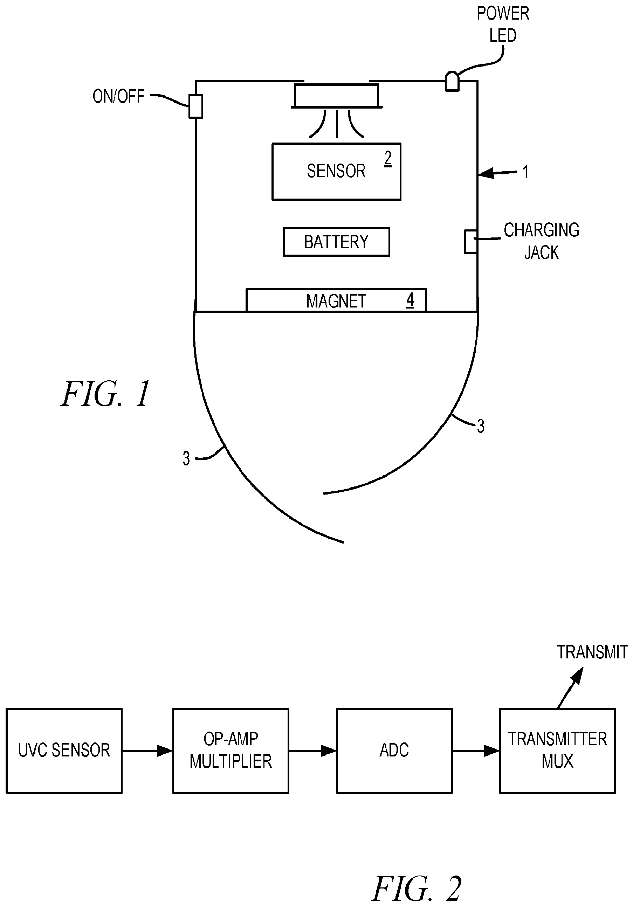

One aspect of the present disclosure pertains to UVC sensors that can optionally be positioned at various locations in a room with surfaces to be decontaminated to sense the level of exposure to UVC light. One example of a UVC sensor 1 is shown in Fig. 1. The UVC sensor 1 includes an optical sensor 2 that senses UVC light 3 and transmits a signal indicative of the intensity, power output, or other property of UVC light indicative of the sterilization effectiveness of that UVC light to which the sensor 2 is exposed. The optical sensor 2 can be positioned at any suitable location where it is expected to be exposed to the UVC light transmitted by a UVC source, or where it is necessary to determine the exposure of UVC light. In some embodiments, the UVC sensor can be sensitive to only a specific, narrow band of UVC frequencies. Furthermore, it is desirable that the UVC sensor is capable of sensing UVC light at large incidence angles such that the sensor does not need to be directly in line of site of the UVC source.

In some embodiments, hook and loop straps 3, a magnetic mount 4, the like, or some combination thereof may be provided in order to mount the UVC sensors 1 in a variety of locations and to a variety of objects. In various embodiments, the UVC sensors 1 may be temporarily mounted to test the efficacy of an irradiation protocol for a room layout. However, in other embodiments, it may be desirable to permanently mount the UVC sensors 1. Depending on whether a UVC sensor is temporarily or permanently mounted, it may be desirable to have a removable, rechargeable, or wired power source such as a lithium ion battery. Further in this vein, Fig. 1 indicates an on/off power switch and power LED indicator such that the UVC sensors can be turned on only during an irradiation process.

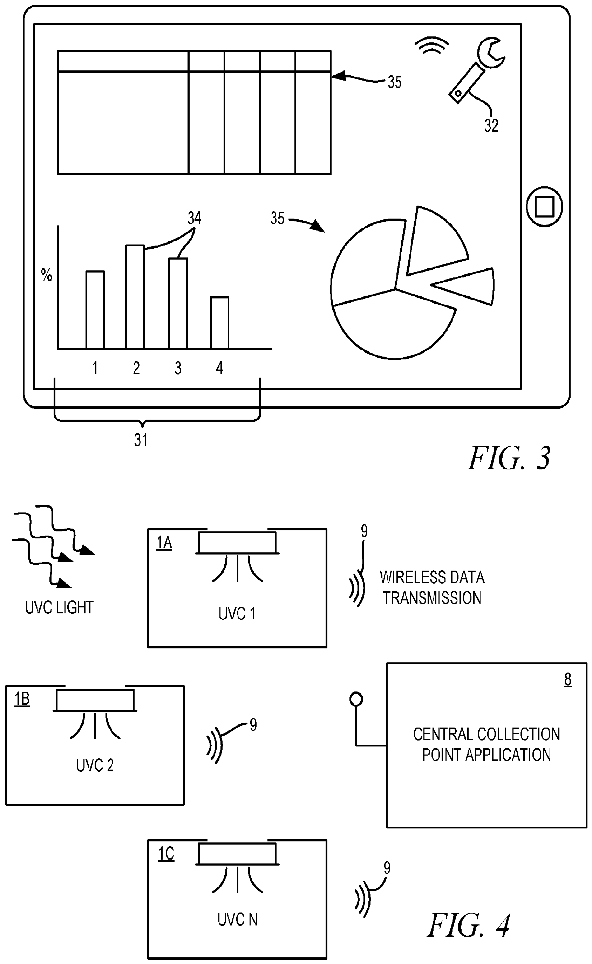

Fig. 2 illustrates a flow chart of the UVC sensor 1 operation. According to the flow chart, a signal from the optical sensor 2 is passed to an opamp multiplier 5. Next, the signal is converted to a digital signal by an analog to digital converter 6, before the signal is multiplexed and transmitted by a transmitter 7. As further illustrated in Fig. 4, multiple UVC sensors 1A, 1B, 1C may work together to generate and report data related to UVC irradiation. In some embodiments, each UVC sensor 1A, 1B, 1C may transmit, via a wireless communication channel 9 (e.g., IEEE 802.11, Bluetooth, etc.) data directly to a tablet 8 or similar personal computing device, which can generate a graphical representation of the collected data for convenient viewing by a clinician. However, in other embodiments, the UVC sensors may transmit data to a central collection point. In such an embodiment, the sensors may only transmit data to the central collection point in an industrial, scientific, and medical (ISM) band. In this way, transmissions from the UVC sensors can be on the order of megahertz, thereby saving power consumption at the UVC sensor. In contrast, WiFi and Bluetooth technologies, which are often most compatible with personal computing devices, transmit in gigahertz bandwidths requiring additional power. The central collection point may then collect all of the data from the UVC sensors and forward it to a tablet or other computing device using WiFi or nearfield Bluetooth technologies.

Fig. 3 illustrates an example of how the UVC sensor data may be displayed on a tablet or similar device. In this example, one section of the screen illustrates a bar graph 31 for illustrating the intensity of light measured at each UVC sensor. Each bar 34 on the bar graph 31 can represent a UVC sensor. The bars may change color (e.g., green to yellow to red) indicating whether the detected UVC is at an adequate level. In some embodiments, the bars 34 are customizable. For example, a first bar may represent a UVC sensor placed on a bed requiring one level of UVC irradiation. Another bar may represent a sensor placed on a wall across the room requiring a second level of UVC irradiation. Accordingly, the color coding of each bar may be specific to the required customized irradiation levels required. A table section 35 may indicate a current irradiation level for each sensor and/or an irradiation intensity accumulation over time (i.e., shown in Joules or Watts). Also shown is a pie chart section 35 for showing similar data. Of course, the above represents but a few examples of what data may be illustrated and how that data be illustrated, but does not represent a limiting embodiment. Other data collected by the UVC sensor may be displayed in various formats as known to those skilled in the art. The application for displaying the UVC sensor data may also comprise a settings screen (accessible by button 32, but not otherwise shown). As discussed above, in many embodiments the application on the computing device interfaces with the UVC sensors and/or the central collection point using WiFi or Bluetooth technologies, but any communication technology is envisioned within the scope of the present disclosure.

The embodiments described above utilize an array of sensors to detect the extent to which different regions of a room are exposed to UVC light emitted by a stationary UVC source. In addition to displaying data, it is also envisioned that the UVC sensors could be used to automatically control a motorized UVC source that travels to a plurality of different locations throughout the room. That is, for example, if one UVC sensor detects a less than adequate level of UVC, it could direct the UVC source to autonomously move in such a way that the location of the reporting UVC sensor receives additional irradiation. Other embodiments are also envisioned that use the UVC sensors as a safety mechanism during periods of irradiation. That is, for example, if a UVC sensor detects UVC at a time when no irradiation is supposed to be occurring, it may be used to alert all individuals in the area of the hazardous conditions.

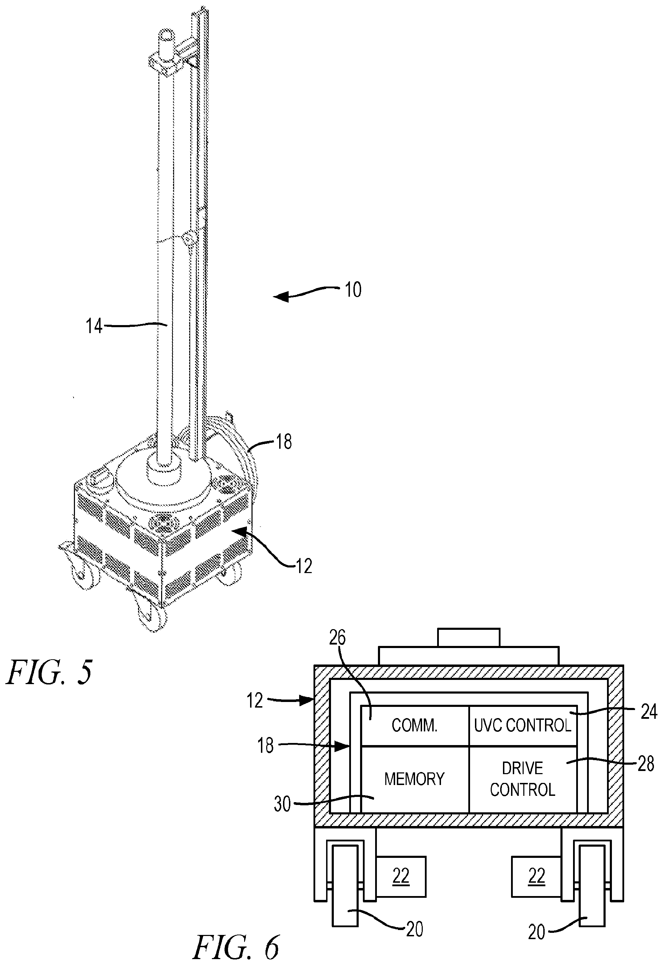

According to alternate embodiments, the UVC source 10 can be mobile, autonomously transported to a plurality of different locations in a room by a motorized base 12 as schematically illustrated in FIGs. 5-7. Transporting the UVC source 10 as described herein allows for the UVC source 10 to expose surfaces to UVC light while the UVC source 10 is at a second location, wherein such surfaces are shielded from the UVC light emitted by the UVC source 10 at a first location (e.g., where the UVC source 10 is initially activated). Transporting the UVC source 10 to a plurality of different locations throughout the room allows for the UVC-emitting bulbs 14 to be positioned within close proximity (e.g., within five (5) feet, or optionally within four (4) feet, or optionally within three (3) feet, or optionally within two (2) feet, or optionally within one (1) foot) to the surfaces being decontaminated. Thus, a plurality of UVC-emitting bulbs 14 that are relatively low power (e.g., unable to achieve a desired level of pathogen reduction such as a 1 log₁₀ reduction on the surfaces in under ten (10) minutes when separated from the surfaces by three (3) feet), can be included as part of the UVC source 10 instead of a central, high-power UVC bulb that broadcasts UVC light from a single location within the room to achieve pathogen reduction.

As shown in FIG. 5, the UVC source 10 is not stationary, but mobile. The UVC source 10 includes at least one, and optionally a plurality of vertically-oriented UVC-emitting bulbs 14 extending upwardly from the base 12. An electric cord 16 is configured to be plugged at one end into a conventional AC mains wall outlet supplied with electric energy from a public utility, for example, and is operatively connected to conduct electric energy from the outlet to a controller 18 (schematically shown in FIG. 6) within the base 12. A plurality of wheels 20 are coupled to the underside of the base 12, allowing the base 12, and accordingly the UVC source 10, to be rolled along the surface of a floor. At least one, and optionally a plurality of the wheels 20 can optionally be pivotally coupled to the underside of the base 12 to allow the UVC source 10 to be rolled around corners and otherwise change directions of travel. According to alternate embodiments, the wheels 20 can optionally be replaced by any suitable transportation devices such as one or a plurality of motor-driven tracks that include a belt that travels over a plurality of wheels, allowing the base 12 to be skid steered, for example. However, for the sake of brevity and clarity, the embodiments utilizing wheels 20 to transport the base 12 will be described in detail.

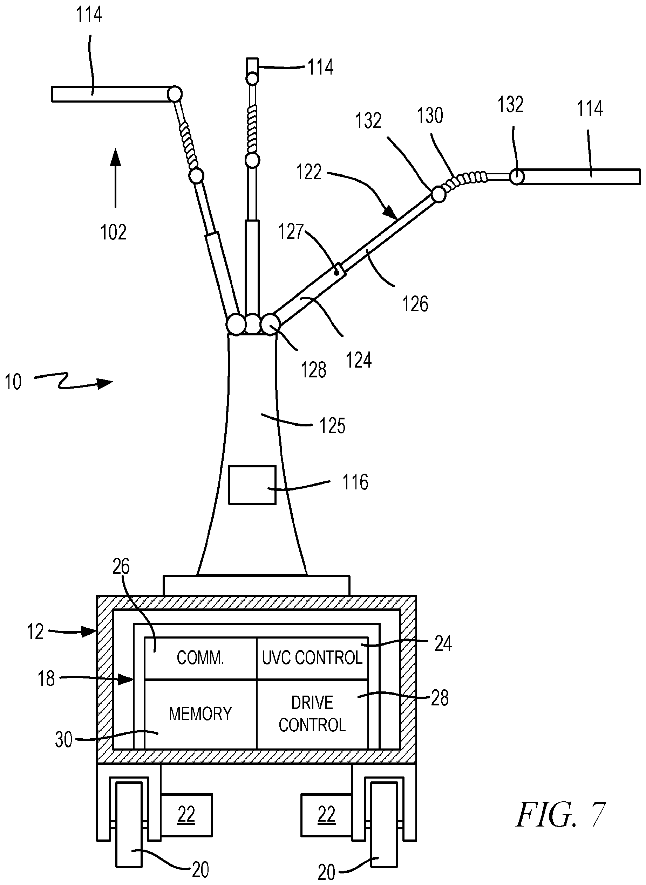

Instead of, or in addition to the vertically-oriented UVC-emitting bulbs 14, another embodiment of the UVC source 10 includes a plurality of independently-positionable sources including one or a plurality of UVC bulbs 114, as shown in FIG. 7, that each direct UVC light toward the surface(s) to be rendered pathogen reduced. The UVC bulbs 114 are said to be independently positionable in that the position of each can be adjusted and maintained relative to the other UVC bulb(s) 114. Such a UVC source 10 can also optionally include an occupant sensor that determines whether the room 1 is occupied or not, and a controller 116 that interferes with emission of the UVC light if the room 1 is, or becomes occupied based on a signal from the occupant sensing system.

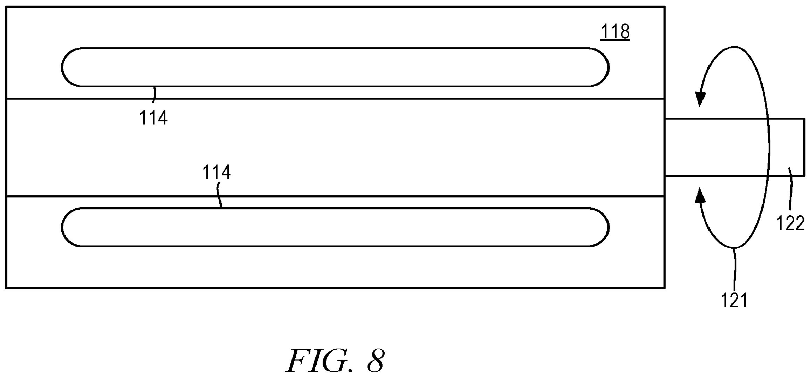

From the viewpoint illustrated in FIG. 8 looking up into a pair of parallel bulbs 114 in the direction indicated by arrow 102 in FIG. 7, the bulb(s) 114 are coupled to a reflective shield 118 that focuses the UVC light toward the surface(s) being decontaminated. The bulb 114 and/or reflective shield 118 can be pivotally coupled to a distal end of an articulated arm 122 or other suitable support that allows the bulbs 14 and/or shield 118, to be pivoted about a rotational axis in the directions indicated by arrow 121 and otherwise positioned in a suitable position relative to the surface to achieve the desired level of decontamination within a predetermined period of time (e.g., less than ten (10) minutes, less than eight (8) minutes, less than six (6) minutes, less than 4 (4) minutes, less than three (3) minutes, etc.), once activated. The parallel bulbs 114 provided with a common shield 118 are independently positionable relative to the bulb(s) 114 supported by the other arm(s) 122. An example of such repositionable bulbs is described in U.S. Patent No. 9,095,633

According to the embodiment in FIG. 7, each arm 122 has a portion including an adjustable length extending generally away from a base portion 125, which can be facilitated by an external member 124 that telescopically receives an internal member 126, or other suitable length adjustment mechanism (e.g, sliding track, etc...). A locking member 127 such as a spring-biased pin urged toward a locking position, etc... can be provided to one or both of the external and internal members 124, 126 to maintain a desired length of the arm 122, once manually established. A hinge 128 or other connector suitable to allow angular adjustment of the arm 122 relative to the base 125 can be disposed between the base 125 and the arm 122. A bendable joint 130 can also be provided anywhere along the length of the arm 122, such as adjacent to the distal end of the arm 122 where the bulb(s) 114 is/are supported. The joint 130 can be formed from a plastically-deformable flexible material that can be manually bent to position the bulb(s) 114, yet be sufficiently rigid to maintain the position of the housing relative to the arm 122 once the bending force has been removed. Further, a hinge 132 can also optionally be positioned along the arm 122 before and/or after the joint 130 to allow further adjustment of the position of the bulb(s) 114 to achieve the desired coverage of the surface to be decontaminated with UVC light. As with any of the hinges described herein, the hinge(s) 132 can be selectively lockable, meaning a locking member such as a set screw, for example, can be loosened to allow the structures coupled to opposite sides of the hinge(s) 132 to be pivotally adjusted relative to each other. Once the desired adjustment has been completed, the set screw or other locking member can be tightened to interfere with further pivotal adjustment of the structures relative to each other.

The base 125 supports the arms 122 at a desired elevation above the floor of the room. The base 125 supports the controller 116 that can be manipulated by a user to control operation of the UVC source 10 (e.g., independently control operation of each bulb 114 to emit UVC light, optionally to cause one bulb 114 to remain energized longer than another one of the bulbs 114), and optionally houses an on-board power supply such as a rechargeable battery bank or circuitry for utilizing electricity from an AC mains source such as a wall outlet supplied by an electric utility that can be used to energize the bulbs 114 and power the controller 116. A power cord can be plugged into an AC mains electric outlet supplied by an electric power utility to obtain the electric energy needed to power the UVC source 10.

Regardless of the embodiment of the UVC source 10, at least one, and optionally each of the plurality of wheels 20 can be driven by an electric motor 22 to allow the UVC source 10 to travel autonomously, without the direct assistance of a human user while the UVC source 10 is underway. In other words, the UVC source 10 can navigate a room being decontaminated to render that room pathogen reduced without being physically contacted by a human user to steer the UVC source 10 during the decontamination process, and optionally without receiving remote control signals being manually input in real-time by a human operator.

To be rendered "pathogen reduced", at least a portion, optionally less than all, of a biologically-active present on the exposed surface of objects exposed to the UVC light emitted by the UVC bulb(s) 14 is deactivated. For instance, rendering objects in a room pathogen reduced does not necessarily require those objects to be made 100% sterile, free of any and all biologically-active organisms that can viably infect a human being. Instead, being rendered pathogen reduced requires a lower level of biologically-active contagions viable to cause an infection to remain on the surface of the objects after performance of the decontamination process herein than existed on those surfaces prior to performance of the decontamination process. Also, deactivation of the biologically-active contagions can include killing live contagions, or at least neutralizing their ability (e.g., rendering them no longer viable) to reproduce to an extent that results in an infection in a human exposed to the deactivated contagions.

According to other embodiments, decontaminated surfaces can be required to possess a lower level of viable or otherwise biologically-active contagions than a threshold quantity permitted under U.S. Food and Drug Administration requirements on objects dedicated for use in a sterile field such as in an operating room during a surgical procedure. According to other embodiments, the decontamination process can be required to kill or otherwise deactivate at least 99% of all living or otherwise biologically-active contagions present on the exposed surfaces immediately prior to performance of the decontamination process to render those surfaces pathogen reduced.

According to yet other embodiments, achieving pathogen reduction amounting to a high-level disinfection of the surfaces in the room utilizing the UVC source 10 can involve deactivation of a suitable portion of the biologically-active contagions to achieve at least a 1 log₁₀ reduction of viable contagions on the object that remain infectious (i.e., no more than 1/10th of the biologically-active contagions originally present remain active or infectious at a time when the decontamination process is completed). According to yet other embodiments, achieving high-level disinfection of the surfaces utilizing the UVC source 10 can involve deactivation of a suitable portion of the biologically-active contagions to achieve at least a 3 log₁₀ reduction (i.e., 1/1,000th) of viable contagions originally present on the surfaces exposed to UVC light. According to yet other embodiments, achieving high-level disinfection of such surfaces can involve deactivation of a suitable portion of the biologically-active contagions to achieve at least a 5 log₁₀ reduction (i.e., 1/100,000th) of viable contagions thereon.

As shown in FIG. 6, an independently-controllable electric motor 22 is provided to each of the wheels 20, however alternate embodiments can include a plurality of wheels 20 driven by a common electric motor 22 through the use of a drivetrain (not shown). Yet other embodiments can include a steering mechanism (not shown) for controlling a direction in which the UVC source 10 travels, that allows less than all of the wheels 20 to be driven by a motor. But for purposes of this disclosure, each of the wheels 20 is driven such that the direction in which the UVC source 10 travels can be controlled by selectively controlling operation of each of the motors 22 individually, at different speeds. Thus, when a first motor 22 is operated to drive its respective wheel at one speed, and a second motor 22 on an opposite side of the base 12 drives its respective wheel at a faster speed, then the UVC source 10 is caused to turn toward the slower-driven wheel 20.

A schematic representation of the controller 18 is shown in FIG. 6. For the illustrated embodiment, the controller 18 includes a UVC control component 24 that selectively controls the delivery of electric energy supplied via the power cord 16 to the UVC-emitting bulb(s) 14. The UVC control component 24 can include a timer that, upon expiration of a predetermined period of time, which can optionally be manually specified by a user, causes deactivation of the UVC-emitting bulb(s)14.

According to alternate embodiments, one or more of the UVC sensors described above can optionally communicate, in real-time with an optional communication component 26 provided to the controller 18 to limit the duration of a decontamination process during which the UVC-emitting bulb(s) 14 is/are activated. For example, a plurality of the UVC sensors can be arranged in a room that is to be decontaminated utilizing the UVC source 10. The UVC source 10 can be placed in the same room and activated in a mode that maintains operation of the UVC bulb(s) 14 until all of the UVC sensors therein have been exposed to a threshold minimum level of UVC light emitted by the UVC source 10. Each UVC sensor measures the extent of UVC exposure and, in response to sensing exposure to the minimum level of UVC light, transmits a wireless signal to be received by the communication component 26. Once all of the UVC sensors have transmitted such a signal indicating adequate exposure to UVC light for the decontamination process and the signals are received by the communication component 26, the communication component 26 transmits a signal to the UVC control component 24 which, in turn, deactivates the UVC bulb(s) 14.

Alternate embodiments of the communication component 26 can optionally receive signals that are used to control relocation of the UVC source 10 using the wheels 20, as described below. For instance, the UVC sensors described above as being distributed throughout a room can optionally emit signals indicative of the level of UVC light to which those UVC sensors have been exposed. Such signals can be received by the communication component 26 and utilized by the communication component 26 to determine whether there are UVC sensors within the room that have not been exposed to a sufficient level of UVC light to achieve the desired level of decontamination within regions adjacent to the UVC sensors. Based, at least in part on such a determination, the UVC source 10 can remain within close proximity to the insufficiently-exposed UVC sensors until those sensors have been exposed to a suitable level of UVC light to achieve the desired level of decontamination before proceeding to a subsequent location.

The controller 18 can also include a drive control component 28 that controls operation of the electric motor(s) 22 driving the wheels 20 based on a plurality of waypoints stored by a computer-readable memory forming a portion of a memory component 30. Each waypoint establishes a location within a room or other environment where the UVC source 10 is to arrive autonomously as part of its journey during a decontamination process. The waypoints can optionally be saved by the memory component to reflect a generic pattern common to a plurality of patient rooms within a hospital, guest rooms in a hotel, or other commonly-configured locations. Thus, the UVC source 10 can be placed at a starting point common to each such room and activated in a decontamination mode that calls for the UVC source 10 to travel to each waypoint autonomously to complete the decontamination process. Once the decontamination process is complete in one such commonly-configured room, the UVC source 10 can be manually transported to the next commonly-configured room, placed at the starting point and reactivated in that mode to also decontaminate that room. This process can be repeated for each such commonly-configured room to be decontaminated. The memory component 30 can optionally store different waypoints for different room configurations, allowing an operator to press a button specific to a given room to cause the UVC source 10 to autonomously navigate the waypoints specific to the button that was pressed.

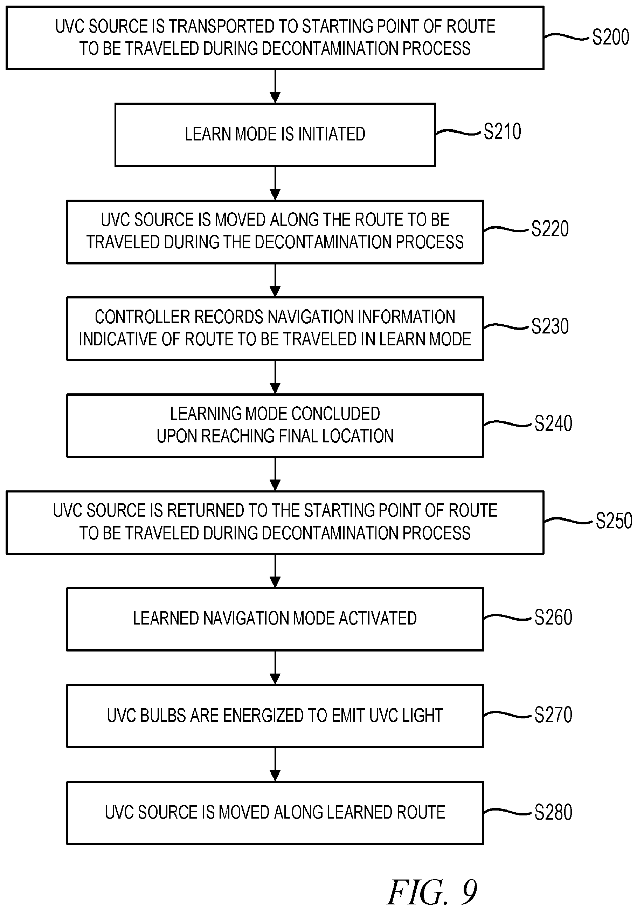

The UVC source 10 can be placed in a "learn" mode to allow an operator to manually enter the desired waypoints for a specific room into the memory component 30. In use, as illustrated by the flow diagram of FIG. 9, the operator can manually transport (e.g., push or otherwise directly control) the UVC source 10 to the starting point of a route to be navigated by the UVC source 10 to complete a decontamination process at step S200. Once at the starting point, the operator can cause the UVC source 10 to enter the learn mode at step S210 via an appropriate user interface, and then manually move the UVC source 10, at step S220, along the route to be autonomously traveled by the UVC source 10 with the UVC-emitting bulb(s) 14 energized after the operator has left the room. The drive control 28 can include one or more sensors that can be used to sense signals indicative of a heading (e.g., angular pivoting of the wheels 20 to determine a direction relative to the starting point) and the distance traveled (e.g., a timer that determines the duration for which the motor 22 would be operational to travel between waypoints) until the final location is reached, as manually indicated by the operator. This navigation information can be recorded by the controller 18 at step S230.

Upon reaching the final location to which the UVC source 10 will travel as part of the decontamination process, the operator can identify this location by terminating the learn mode via an appropriate user interface at step S240. To conduct the decontamination process, the operator can manually return the UVC source 10 to the starting point at step S250, optionally arrange one or a plurality of UVC sensors throughout the room at desired locations to ensure a thorough decontamination, and initiate the decontamination process at step S260 by selecting the learned navigation mode via an appropriate user interface. Following the expiration of a predetermined period of time sufficient to allow the operator to exit the room and close the door, the UVC control component 24 activates the UVC-emitting bulb(s) 14 at step S270. Once the desired level of decontamination has been achieved on the surfaces within the room exposed to the UVC light emitted by the UVC bulb(s) 14 with the UVC source 10 in the starting point, the drive control component 28 controls operation of the motor(s) 22 to move the UVC source 10 along the route learned in the learn mode at step S280. Again, movement of the UVC source 10 can be influenced by feedback from one or more of the UVC sensors in the room and received by the communication component 26, by a timer (e.g., after remaining at the starting point for a predetermined period of time, move onward), and/or any other factor indicative of a level of decontamination of surfaces near the starting point. The UVC source 10 can utilize GPS navigational triangulation, a timer and directional sensor to activate the motor(s) 22 for known lengths of time in certain directions, and any other control factors during autonomous transportation of the UVC source 10 along the learned (or preprogrammed) route. The rate at which the UVC source 10 travels can be sufficient to achieve the desired level of decontamination as the UVC source 10 moves, and/or the UVC source 10 can stop at one, a plurality or all of the waypoints learned in the learn mode to achieve the desired level of decontamination of the exposed surfaces. Upon reaching the final destination for that learned route, the UVC bulbs are de-energized to complete the decontamination process.

According to alternate embodiments, hospital rooms, hotel rooms, etc., can optionally be provided with one or more markings on the floor (e.g., a stripe of reflective material, dots of paint, etc...) that can be sensed by a sensor provided to the underside of the base 12. The sensor can be operationally connected to communicate directional signals to the drive control component 28 to cause selective operation of the motor(s) 22 as appropriate to cause the UVC source 10 to follow the path defined by the markings on the floor. According to such embodiments, the markings can eliminate the need to pre-program waypoints into the memory component 30, instead allowing the UVC source 10 to simply follow the markings along a desired path.

For any of the embodiments above where the UVC source 10 is mobile, the base 12 or other portion of the UVC source 10 can optionally be equipped with a proximity sensor that utilizes ultrasonic waves, optical sensors, etc... to detect when any portion of the UVC source 10 approaches a foreign object (e.g., furniture in the room, medical equipment on the floor, etc...) and is about to make physical contact with that foreign object. The proximity sensor can be operatively connected to transmit a signal to the drive control component 28 which, in turn, can deactivate the motor(s) 22 and stop the UVC source 10 before the UVC source 10 actually makes contact with the foreign object. Impending contact with a foreign object can also optionally be grounds to deactivate the UVC-emitting bulb(s) 14, thereby prematurely terminating the decontamination process. Under such circumstances, the operator can optionally be informed of premature termination of the decontamination process by a visible and/or audible indicator provided to the UVC source 10, via a remote control outside of the room being decontaminated in response to receiving a signal transmitted by the communication component 26, simply by the position of the UVC source 10 at the unexpected location where decontamination was prematurely terminated instead of at the known end of the route, or via any other indicator.

Illustrative embodiments have been described, hereinabove. It will be apparent to those skilled in the art that the above devices and methods may incorporate changes and modifications without departing from the the scope of the claims. It is intended to include all such modifications and alterations within the scope of the claims Furthermore, to the extent that the term "includes" is used in either the detailed description or the claims, such term is intended to be inclusive in a manner similar to the term "comprising" as "comprising" is interpreted when employed as a transitional word in a claim.

## Claims

1. Use of a decontamination apparatus for decontaminating beds, tray tables, seats within a room, wherein the decontamination apparatus comprises:
a motorized base comprising a transport system that is operable to move the decontamination apparatus; the transport system comprising a plurality of wheels and a motor that drives at least one of the plurality of wheels;
a plurality of UVC bulbs that are supported by the base, wherein each of the UVC bulbs is configured to emit UVC light; and
a controller configured to store a route to be traveled by the decontamination apparatus from a starting point to a destination during a decontamination process and to control operation of the transport system to move the decontamination apparatus along the route, the controller being configured for:
1) entering a learn mode caused by an operator via a user interface once the apparatus is manually transported to a starting point by the operator,
2) recording navigation information along the route when the apparatus is manually moved by the operator along said route from the starting point to the destination,
3) exiting the learn mode caused by the operator via the user interface when the destination is reached,
4) activating navigation mode caused by the operator via the user interface once the apparatus is manually returned to the starting point,
5) activating UVC bulbs to emit UVC light to start a decontamination process,
6) moving the apparatus along the route according to the recorded navigation information,
wherein the controller comprises a drive control component controlling operation of the motor based on a plurality of waypoints stored by a computer readable memory of a memory component of the apparatus;
wherein, when in the learn mode, the controller is configured for receiving the plurality of waypoints manually entered by the operator.

2. The use of claim 1, wherein the memory component is configured to save waypoints reflecting a pattern of one room or common to a plurality of rooms.

3. The use of claim 1, wherein the memory component is configured to save different waypoints for different room configurations.

4. The use of any of claims 1 to 3, wherein the drive control comprises one or more sensors configured to sense signals indicative of a heading and a distance traveled until the destination is reached.

5. The use of any of claims 1 to 4, wherein an UVC control component of the controller is configured to activate the UVC bulbs following the expiration of a time, in order to allow the operator to exit the room.

6. The use of any of claims 1 to 5, wherein the apparatus comprises a communication device and a plurality of UVC sensors to be arranged at locations in the room, wherein movement of the transport system is influenced by feedback from one or more of the UVC sensors in the room received by the communication device.

7. The use of any of claims 1 to 6, wherein the controller is configured to stop the apparatus at one, a plurality or all the waypoints along the route during the decontamination process to achieve decontamination of exposed surfaces

## Patentansprüche

1. Verwendung einer Dekontaminationsvorrichtung zum Dekontaminieren von Betten, Tablett-Tischen, Sitzen in einem Raum, wobei die Dekontaminationsvorrichtung umfasst:
eine motorisierte Basis, welche ein Transportsystem umfasst, welches betriebsfähig ist, um die Dekontaminationsvorrichtung zu bewegen; wobei das Transportsystem eine Mehrzahl von Rädern und einen Motor umfasst, welcher wenigstens eines der Mehrzahl von Rädern antreibt;
eine Mehrzahl von UVC-Birnen, welche durch die Basis gehaltert sind, wobei jede der UVC-Birnen dazu eingerichtet ist, UVC-Licht zu emittieren; und
eine Steuereinrichtung, welche dazu eingerichtet ist, einen durch die Dekontaminationsvorrichtung zurückzulegenden Weg von einem Startpunkt zu einem Ziel während eines Dekontaminationsprozesses zu speichern und einen Betrieb des Transportsystems zu steuern, um die Dekontaminationsvorrichtung entlang des Wegs zu bewegen, wobei die Steuereinrichtung eingerichtet ist zum:
1) Eintreten in einen Lernmodus, welcher durch einen Bediener über eine Benutzerschnittstelle hervorgerufen wird, sobald die Vorrichtung durch den Bediener manuell zu einem Startpunkt transportiert wird,
2) Aufzeichnen von Navigationsinformationen entlang des Wegs, wenn die Vorrichtung durch den Bediener manuell entlang des Wegs von dem Startpunkt zu dem Ziel bewegt wird,
3) Verlassen des Lernmodus, welcher durch den Bediener über die Benutzerschnittstelle hervorgerufen wird, wenn das Ziel erreicht ist,
4) Aktivieren eines Navigationsmodus, welcher durch den Bediener über die Benutzerschnittstelle hervorgerufen wird, sobald die Vorrichtung manuell zu dem Startpunkt zurückgebracht ist,
5) Aktivieren von UVC-Birnen, um UVC-Licht zu emittieren, um einen Dekontaminationsprozess zu starten,
6) Bewegen der Vorrichtung entlang des Wegs gemäß den aufgezeichneten Navigationsinformationen,
wobei die Steuereinrichtung eine Antriebssteuerungskomponente umfasst, welche einen Betrieb des Motors auf Grundlage einer Mehrzahl von durch einen computerlesbaren Speicher einer Speicherkomponente der Vorrichtung gespeicherten Wegpunkten steuert;
wobei, wenn sie sich in dem Lernmodus befindet, die Steuereinrichtung dazu eingerichtet ist, die Mehrzahl von Wegpunkten zu empfangen, welche manuell durch den Bediener eingegeben worden sind.

2. Verwendung nach Anspruch 1, wobei die Speicherkomponente dazu eingerichtet ist, Wegpunkte zu sichern, welche ein Muster eines Raums oder gemeinsam für eine Mehrzahl von Räumen reflektieren.

3. Verwendung nach Anspruch 1, wobei die Speicherkomponente dazu eingerichtet ist, verschiedene Wegpunkte für verschiedene Raumkonfigurationen zu sichern.

4. Verwendung nach einem der Ansprüche 1 bis 3, wobei die Antriebssteuereinrichtung einen oder mehrere Sensoren umfasst, welche dazu eingerichtet sind, Signale zu erfassen, welche für einen Kurs und eine zurückgelegte Strecke indikativ sind, bis das Ziel erreicht ist.

5. Verwendung nach einem der Ansprüche 1 bis 4, wobei eine UVC-Steuerungskomponente der Steuereinrichtung dazu eingerichtet ist, auf den Ablauf einer Zeit folgend, die UVC-Birnen zu aktivieren, um dem Bediener zu erlauben, den Raum zu verlassen.

6. Verwendung nach einem der Ansprüche 1 bis 5, wobei die Vorrichtung eine Kommunikationsvorrichtung und eine Mehrzahl von an Stellen in dem Raum anzuordnenden UVC-Sensoren umfasst, wobei eine Bewegung des Transportsystems durch eine durch die Kommunikationsvorrichtung empfangene Rückmeldung von einem oder mehreren der UVC-Sensoren in dem Raum beeinflusst wird.

7. Verwendung nach einem der Ansprüche 1 bis 6, wobei die Steuereinrichtung dazu eingerichtet ist, die Vorrichtung während des Dekontaminationsprozesses an einem, einer Mehrzahl oder all der Wegpunkte entlang des Wegs zu stoppen, um eine Dekontamination exponierter Flächen zu erreichen.

## Revendications

1. Utilisation d'un appareil de décontamination pour décontaminer des lits, des tablettes, des sièges à l'intérieur d'une pièce, l'appareil de décontamination comprenant :
une base motorisée comprenant un système de transport qui peut fonctionner pour déplacer l'appareil de décontamination ; le système de transport comprenant une pluralité de roues et un moteur qui entraîne au moins l'une de la pluralité des roues ;
une pluralité d'ampoules UVC qui sont supportées par la base, chacune des ampoules UVC étant conçue pour émettre de la lumière UVC ; et
un dispositif de commande conçu pour stocker un trajet à parcourir par l'appareil de décontamination depuis un point de départ vers une destination durant un processus de décontamination et pour commander le fonctionnement du système de transport pour déplacer l'appareil de décontamination le long du trajet, le dispositif de commande étant conçu pour :
1) entrer dans un mode d'apprentissage causé par un opérateur par l'intermédiaire d'une interface utilisateur une fois que l'appareil est manuellement transporté jusqu'à un point de départ par l'opérateur,
2) enregistrer des informations de navigation le long du trajet lorsque l'appareil est déplacé manuellement par l'opérateur le long dudit trajet depuis le point de départ vers la destination,
3) sortir du mode d'apprentissage causé par l'opérateur par l'intermédiaire de l'interface utilisateur lorsque la destination est atteinte,
4) activer le mode navigation causé par l'opérateur par l'intermédiaire de l'interface utilisateur une fois que l'appareil est manuellement revenu au point de départ,
5) activer des ampoules UVC pour émettre de la lumière UVC pour démarrer un processus de décontamination,
6) déplacer l'appareil le long du trajet en fonction des informations de navigation enregistrées,
le dispositif de commande comprenant un composant de commande d'entraînement commandant le fonctionnement du moteur sur la base d'une pluralité de points de cheminement stockés par une mémoire lisible par ordinateur d'un composant mémoire de l'appareil ;
lorsque dans le mode d'apprentissage, le dispositif de commande est conçu pour recevoir la pluralité des points de cheminement manuellement saisis par l'opérateur.

2. Utilisation selon la revendication 1, le composant mémoire étant conçu pour sauvegarder des points de cheminement reflétant un profil d'une pièce ou communs à une pluralité de pièces.

3. Utilisation selon la revendication 1, le composant mémoire étant conçu pour sauvegarder différents points de cheminement pour différentes configurations de pièce.

4. Utilisation selon l'une quelconque des revendications 1 à 3, la commande d'entraînement comprenant un ou plusieurs capteurs conçus pour détecter des signaux indicatifs d'un cap et d'une distance parcourue jusqu'à ce que la destination soit atteinte.

5. Utilisation selon l'une quelconque des revendications 1 à 4, un composant de commande d'UVC du dispositif de commande étant conçu pour activer les ampoules UVC suite à l'expiration d'un temps, afin de permettre à l'opérateur de sortir de la pièce.

6. Utilisation selon l'une quelconque des revendications 1 à 5, l'appareil comprenant un dispositif de communication et une pluralité de capteurs d'UVC à disposer à des emplacements dans la pièce, le mouvement du système de transport étant influencé par le feedback provenant de l'un ou de plusieurs des capteurs d'UVC dans la pièce reçue par le dispositif de communication.

7. Utilisation selon l'une quelconque des revendications 1 à 6, le dispositif de commande étant conçu pour stopper l'appareil au niveau de l'un, d'une pluralité ou de tous les points de cheminement le long du trajet durant le processus de décontamination pour atteindre la décontamination des surfaces exposées.
